# EUROPEAN PATENT APPLICATION

(11) **EP 4 640 844 A1**
(43) Date of publication of application: **29.10.2025**
(21) Application number: 23907066.7
(22) Date of filing: 19.12.2023
(51) Int. Cl.: C12Q 1/6851, C12N 15/09, C12Q 1/6869, G01N 33/50

(54) **METHOD FOR TESTING POSSIBILITY OF PREGNANCY AND/OR POSSIBILITY OF CHILDBIRTH RESULTING FROM SAID PREGNANCY**

(30) Priority: 21.12.2022 JP 2022204495
(71) Applicant: NATIONAL UNIVERSITY CORPORATION TOKAI NATIONAL HIGHER EDUCATION AND RESEARCH SYSTEM, Nagoya-shi, Aichi 464-8601 (JP)
(72) Inventor: YOKOI, Akira, Nagoya-shi, Aichi 464-8601 (JP); MURAKAMI, Mayuko, Nagoya-shi, Aichi 464-8601 (JP); YOSHIDA, Kosuke, Nagoya-shi, Aichi 464-8601 (JP); OSUKA, Satoko, Nagoya-shi, Aichi 464-8601 (JP); KAJIYAMA, Hiroaki, Nagoya-shi, Aichi 464-8601 (JP)
(74) Representative: Hoffmann Eitle
(86) International application number: PCT/JP2023/045583
(87) International publication number: WO 2024/135706

(57) **Abstract**

Provided is technology for testing the likelihood of pregnancy and/or the likelihood of childbirth resulting from the pregnancy. Provided is a method of testing the likelihood of pregnancy from an egg that had been contained in a follicular fluid collected from a subject and/or the likelihood of childbirth resulting from the pregnancy, including step (1) of detecting at least one miRNA selected from (a) the group consisting of miR-16-2-3p, miR-378a-3p, miR-483-5p, miR-1246, and miR-1290, and/or from (b) the group consisting of miR-204-3p, miR-1468-5p, miR-424-5p, miR-30e-5p, miR-146a-5p, miR-202-5p, miR-370-3p, miR-506-3p, miR-3184-3p, miR-423-5p, miR-365a-5p, miR-514a-3p, miR-92b-5p, miR-27a-5p, miR-6873-3p, miR-451a, miR-122-5p, and miR-122b-3p, in a sample of the follicular fluid.

## Description

### Technical Field

The present invention relates to a method of testing the likelihood of pregnancy and/or the likelihood of childbirth resulting from the pregnancy.

### Background Art

In Japan, 10 to 15% of couples hoping to have children are considered infertile. With the trend of later marriage, the importance of assisted reproductive technology (ART), such as microinsemination and in vitro fertilization, has increased in reproductive medicine. Recently, ART has been approved for insurance coverage, which is expected to further enhance the significance of ART in the future.

The general process of ART is as follows: 1) multiple follicles in the ovaries are matured using an ovulation inducer, and follicular fluid is obtained by puncturing the follicles; 2) eggs are retrieved from the obtained follicular fluid and fertilized with sperm in a culture medium or under a microscope to obtain fertilized eggs; 3) when multiple fertilized eggs are obtained, one or two high-grade fertilized eggs are selected based on visual grading criteria and returned to the uterus; and 4) if high-grade fertilized eggs remain, they may be cryopreserved. From the viewpoint of reducing medical costs and the burden on the mother, selecting eggs with a higher probability of pregnancy is important for conducting ART more efficiently.

In NPL 1, a search for test markers for the possibility of pregnancy was conducted by comparing the expression levels of miRNAs in embryo culture mediums between successful pregnancy and unsuccessful pregnancy. However, this technique requires egg fertilization and embryo culture, thus lacking simplicity.

### Citation List

### Non-patent Literature

NPL 1: Journal of Advanced Research, 2021 Jul; 31: 25-34.

### Summary of Invention

### Technical Problem

An object of the present invention is to provide technology for testing the likelihood of pregnancy and/or the likelihood of childbirth resulting from the pregnancy.

### Solution to Problem

After conducting extensive research in light of the object above, the present inventors discovered that the following method can achieve the object: a method of testing the likelihood of pregnancy from an egg that had been contained in follicular fluid collected from a subject and/or the likelihood of childbirth resulting from the pregnancy, comprising step (1) of detecting at least one miRNA selected from (a) the group consisting of miR-16-2-3p, miR-378a-3p, miR-483-5p, miR-1246, and miR-1290, and/or from (b) the group consisting of miR-204-3p, miR-1468-5p, miR-424-5p, miR-30e-5p, miR-146a-5p, miR-202-5p, miR-370-3p, miR-506-3p, miR-3184-3p, miR-423-5p, miR-365a-5p, miR-514a-3p, miR-92b-5p, miR-27a-5p, miR-6873-3p, miR-451a, miR-122-5p, and miR-122b-3p, in the follicular fluid sample. The inventors conducted further research based on this finding and completed the present invention. Specifically, the present invention encompasses the following aspects.

### Item 1.

A method of testing the likelihood of pregnancy from an egg that had been contained in a follicular fluid collected from a subject and/or the likelihood of childbirth resulting from the pregnancy, comprising step (1) of detecting at least one miRNA selected from (a) the group consisting of miR-16-2-3p, miR-378a-3p, miR-483-5p, miR-1246, and miR-1290, and/or from (b) the group consisting of miR-204-3p, miR-1468-5p, miR-424-5p, miR-30e-5p, miR-146a-5p, miR-202-5p, miR-370-3p, miR-506-3p, miR-3184-3p, miR-423-5p, miR-365a-5p, miR-514a-3p, miR-92b-5p, miR-27a-5p, miR-6873-3p, miR-451a, miR-122-5p, and miR-122b-3p, in a sample of the follicular fluid.

### Item 2.

The method according to Item 1, wherein the miRNA includes at least one miRNA selected from the group consisting of miR-16-2-3p, miR-378a-3p, miR-483-5p, miR-1246, and miR-1290.

### Item 3.

The method according to Item 1, wherein the miRNA includes at least one miRNA selected from the group consisting of miR-16-2-3p, miR-378a-3p, and miR-483-5p.

### Item 4.

The method according to Item 1, wherein the miRNA includes miR-16-2-3p, miR-378a-3p, and miR-483-5p.

### Item 5.

The method according to Item 1, wherein the sample of the follicular fluid is a cell-reduced fraction of the follicular fluid.

### Item 6.

The method according to Item 1, wherein the sample of the follicular fluid is an extracellular vesicle purified from the follicular fluid.

### Item 7.

The method according to Item 1, wherein the miRNA includes at least one miRNA selected from the group consisting of miR-204-3p, miR-1468-5p, miR-424-5p, miR-16-2-3p, miR-30e-5p, miR-1246, miR-1290, miR-378a-3p, miR-483-5p, miR-146a-5p, miR-202-5p, miR-370-3p, miR-506-3p, miR-3184-3p, miR-423-5p, miR-365a-5p, miR-514a-3p, miR-92b-5p, miR-27a-5p, and miR-6873-3p.

### Item 8.

The method according to Item 1, further comprising step (2) of determining the likelihood of pregnancy from the egg and/or the likelihood of childbirth resulting from the pregnancy on the basis of a level or concentration of the miRNA detected in step (1).

### Item 9.

The method according to Item 8,
wherein
step (2) comprises at least one step selected from the group consisting of step (2a) and step (2b),
   wherein
step (2a) determines that the likelihood of pregnancy from the egg and/or the likelihood of childbirth resulting from the pregnancy is high when the level or concentration of at least one miRNA selected from the group consisting of miR-1468-5p, miR-424-5p, miR-16-2-3p, miR-30e-5p, miR-146a-5p, miR-202-5p, and miR-451a detected in step (1) is equal to or higher than a cutoff value, and/or determines that the likelihood of pregnancy from the egg and/or the likelihood of childbirth resulting from the pregnancy is low when the level or concentration is equal to or lower than the cutoff value, and
step (2b) determines that the likelihood of pregnancy from the egg and/or the likelihood of childbirth resulting from the pregnancy is high when the level or concentration of at least one miRNA selected from the group consisting of miR-204-3p, miR-1246, miR-1290, miR-378a-3p, miR-483-5p, miR-370-3p, miR-506-3p, miR-3184-3p, miR-423-5p, miR-365a-5p, miR-514a-3p, miR-92b-5p, miR-27a-5p, miR-6873-3p, miR-122-5p, and miR-122b-3p detected in step (1) is equal to or lower than a cutoff value, and/or determines that the likelihood of pregnancy from the egg and/or the likelihood of childbirth resulting from the pregnancy is low when the level or concentration is equal to or higher than the cutoff value.

### Item 10.

The method according to any one of Items 1 to 9, wherein the pregnancy is pregnancy through assisted reproductive technology.

### Item 11.

The method according to any one of Items 1 to 9, wherein the subject is a human.

### Item 12.

A drug for testing the likelihood of pregnancy from an egg of a subject and/or the likelihood of childbirth resulting from the pregnancy, comprising a detection agent for at least one miRNA selected from (a) the group consisting of miR-16-2-3p, miR-378a-3p, miR-483-5p, miR-1246, and miR-1290, and/or from (b) the group consisting of miR-204-3p, miR-1468-5p, miR-424-5p, miR-30e-5p, miR-146a-5p, miR-202-5p, miR-370-3p, miR-506-3p, miR-3184-3p, miR-423-5p, miR-365a-5p, miR-514a-3p, miR-92b-5p, miR-27a-5p, miR-6873-3p, miR-451a, miR-122-5p, and miR-122b-3p.

### Advantageous Effects of Invention

The present invention provides technology for testing the likelihood of pregnancy and/or the likelihood of childbirth resulting from the pregnancy.

### Brief Description of Drawings

Fig. 1 shows the read counts of a marker miRNA selected in Test Example 1 for non-pregnancy cases (left side of the graph) and pregnancy cases (right side of the graph). The name of the marker miRNA and the p-value between groups are shown at the top of the graph.
Fig. 2 is shown in the same manner as Fig. 1.
Fig. 3 is shown in the same manner as Fig. 1.
Fig. 4 is shown in the same manner as Fig. 1.
Fig. 5 is shown in the same manner as Fig. 1.
Fig. 6 is shown in the same manner as Fig. 1.
Fig. 7 is shown in the same manner as Fig. 1.
Fig. 8 is shown in the same manner as Fig. 1.
Fig. 9 is shown in the same manner as Fig. 1.
Fig. 10 is shown in the same manner as Fig. 1.
Fig. 11 is shown in the same manner as Fig. 1.
Fig. 12 is shown in the same manner as Fig. 1.
Fig. 13 is shown in the same manner as Fig. 1.
Fig. 14 is shown in the same manner as Fig. 1.
Fig. 15 is shown in the same manner as Fig. 1.
Fig. 16 is shown in the same manner as Fig. 1.
Fig. 17 is shown in the same manner as Fig. 1.
Fig. 18 is shown in the same manner as Fig. 1.
Fig. 19 is shown in the same manner as Fig. 1.
Fig. 20 is shown in the same manner as Fig. 1.
Fig. 21 is shown in the same manner as Fig. 1.
Fig. 22 is shown in the same manner as Fig. 1.
Fig. 23 is shown in the same manner as Fig. 1.

### Description of Embodiments

In the present specification, the terms "comprising" and "including" encompass the concepts of comprising, including, consisting essentially of, and consisting of.

### 1. Testing Method

In an aspect, the present invention relates to a method of testing the likelihood of pregnancy from an egg that had been contained in follicular fluid collected from a subject and/or the likelihood of childbirth resulting from the pregnancy, comprising step (1) of detecting at least one miRNA selected from (a) the group consisting of miR-16-2-3p, miR-378a-3p, miR-483-5p, miR-1246, and miR-1290, and/or from (b) the group consisting of miR-204-3p, miR-1468-5p, miR-424-5p, miR-30e-5p, miR-146a-5p, miR-202-5p, miR-370-3p, miR-506-3p, miR-3184-3p, miR-423-5p, miR-365a-5p, miR-514a-3p, miR-92b-5p, miR-27a-5p, miR-6873-3p, miR-451a, miR-122-5p, and miR-122b-3p, in a sample of the follicular fluid (which may be referred to as "the testing method of the present invention" in the present specification). The following explains the method.

### 1-1. Step (1)

The testing method of the present invention examines the likelihood of pregnancy from an egg that had been contained in follicular fluid, which is the target for miRNA detection (i.e., an egg contained in the same follicle as that containing the follicular fluid), and/or the likelihood of childbirth resulting from the pregnancy.

"Pregnancy" refers to the state in which a fertilized egg has implanted itself on the lining of the uterus and has become capable of growth, or the state in which a fertilized egg is developing, and the term "pregnancy" is not particularly limited in this regard. The pregnancy is preferably pregnancy continuing up to the middle term, and more preferably pregnancy continuing up to the late term. The method of the present invention can examine the likelihood of pregnancy continuing to mid-term pregnancy (in the case of humans, for example, any time between 16 to 27 weeks of pregnancy) or to late-term pregnancy (in the case of humans, for example, any time between 28 to 40 weeks of pregnancy).

While there are no particular restrictions on the course leading to pregnancy, the testing method of the present invention enables the examination of the likelihood of pregnancy due to assisted reproductive technology (e.g., in vitro fertilization and embryo transfer, microinsemination, and embryo transfer with thawed frozen embryos) because the testing method typically allows for the collection of eggs together with follicular fluid. The method of assisted reproductive technology is not particularly limited, and can be a method according to or based on any known method.

Childbirth is the phenomenon in which a living baby is born, and is not particularly limited in this respect. Childbirth is preferably a birth occurring in late-stage pregnancy, and more preferably a full-term delivery. In the case of a human subject, childbirth refers to, for example, delivery after 28 weeks of pregnancy, more preferably after 32 weeks of pregnancy, even more preferably after 35 weeks of pregnancy, and yet more preferably after 37 weeks of pregnancy.

The subject is the target organism for the testing method of the present invention, and there is no particular restriction on the species of organisms. Examples of species for the subject include various mammalian animals, such as humans, monkeys, mice, rats, dogs, cats, and rabbits, with humans being preferable.

The sample of follicular fluid is not particularly limited as long as the sample is derived from follicular fluid of the subject, and may contain miRNAs. The sample of follicular fluid includes both follicular fluid itself and samples prepared from follicular fluid. The sample prepared from follicular fluid (sample derived from follicular fluid) can be obtained by concentrating and purifying extracellular mRNAs in follicular fluid, specifically such as a cell-reduced fraction of follicular fluid, and preferably extracellular vesicles purified from follicular fluid.

The cell-reduced fraction of follicular fluid can be obtained by reducing or removing cells in follicular fluid through centrifugation or other methods.

Extracellular vesicles are not particularly limited as long as they are membrane vesicles secreted or released from cells. Extracellular vesicles are typically defined as membrane vesicles that carry out intercellular communication locally and systemically by transporting intracellular proteins and genetic information (such as mRNAs and microRNAs) outside the cell. Examples of extracellular vesicles include exosomes, microvesicles, apoptotic bodies, ectosomes, microparticles, and secretory microvesicles. From the viewpoint of test accuracy and other factors, extracellular vesicles are particularly preferably exosomes.

Extracellular vesicles can be purified, isolated, concentrated, etc. from follicular fluid according to or based on a known method. Methods for purifying, isolating, and concentrating extracellular vesicles include, for example, ultracentrifugation (e.g., a pellet-down assay, a sucrose cushion assay, and density gradient centrifugation), an assay using immunoaffinity carriers, gel filtration, field-flow fractionation, and FACS. Operations such as purification, isolation, and concentration of extracellular vesicles can also be performed using commercially available kits. These methods can be used either individually or in a combination of two or more.

The method of collecting follicular fluid is not particularly limited and can be performed according to or based on a known method, such as a method including puncturing a follicle while confirming an internal body image, such as an ultrasound image. Typically, an egg in the same follicle is also collected during follicular fluid collection. The target follicle from which follicular fluid is to be collected is not particularly limited as long as it is a follicle in which an egg has matured to a certain level (preferably a mature follicle). From the viewpoint of the accuracy of the testing method of the present invention, in the case of humans, the follicle is preferably a follicle with a diameter of 16 mm or more, more preferably a follicle with a diameter of 18 mm or more, and even more preferably a follicle with a diameter of 18 to 25 mm. The follicle may be a naturally matured follicle or a follicle matured by drug administration (e.g., follicle-stimulating hormone (FSH)).

In step (1), miRNAs in the sample of follicular fluid collected from the subject are detected. The detection target in step (1) is at least one miRNA selected from (a) the group consisting of miR-16-2-3p, miR-378a-3p, miR-483-5p, miR-1246, and miR-1290, and/or (b) the group consisting of miR-204-3p, miR-1468-5p, miR-424-5p, miR-30e-5p, miR-146a-5p, miR-202-5p, miR-370-3p, miR-506-3p, miR-3184-3p, miR-423-5p, miR-365a-5p, miR-514a-3p, miR-92b-5p, miR-27a-5p, miR-6873-3p, miR-451a, miR-122-5p, and miR-122b-3p. These may be collectively referred to as "target biomarkers" below.

The target biomarkers can be identified according to their base sequences etc. in a known database (e.g., miRBase: http://www.mirbase.org/). For example, in the case of humans, the base sequences are as follows:
>hsa-miR-204-3p MIMAT0022693
   GCUGGGAAGGCAAAGGGACGU (SEQ ID NO: 1).
>hsa-miR-1468-5p MIMAT0006789
   CUCCGUUUGCCUGUUUCGCUG (SEQ ID NO: 2).
>hsa-miR-424-5p MIMAT0001341
   CAGCAGCAAUUCAUGUUUUGAA (SEQ ID NO: 3).
>hsa-miR-16-2-3p MIMAT0004518
   CCAAUAUUACUGUGCUGCUUUA (SEQ ID NO: 4).
>hsa-miR-30e-5p MIMAT0000692
   UGUAAACAUCCUUGACUGGAAG (SEQ ID NO: 5).
>hsa-miR-1246 MIMAT0005898
   AAUGGAUUUUUGGAGCAGG (SEQ ID NO: 6).
>hsa-miR-1290 MIMAT0005880
   UGGAUUUUUGGAUCAGGGA (SEQ ID NO: 7).
>hsa-miR-378a-3p MIMAT0000732
   ACUGGACUUGGAGUCAGAAGGC (SEQ ID NO: 8).
>hsa-miR-483-5p MIMAT0004761
   AAGACGGGAGGAAAGAAGGGAG (SEQ ID NO: 9).
>hsa-miR-146a-5p MIMAT0000449
   UGAGAACUGAAUUCCAUGGGUU (SEQ ID NO: 10).
>hsa-miR-202-5p MIMAT0002810
   UUCCUAUGCAUAUACUUCUUUG (SEQ ID NO: 11).
>hsa-miR-370-3p MIMAT0000722
   GCCUGCUGGGGUGGAACCUGGU (SEQ ID NO: 12).
>hsa-miR-506-3p MIMAT0002878
   UAAGGCACCCUUCUGAGUAGA (SEQ ID NO: 13).
>hsa-miR-3184-3p MIMAT0022731
   AAAGUCUCGCUCUCUGCCCCUCA (SEQ ID NO: 14).
>hsa-miR-423-5p MIMAT0004748
   UGAGGGGCAGAGAGCGAGACUUU (SEQ ID NO: 15).
>hsa-miR-365a-5p MIMAT0009199
   AGGGACUUUUGGGGGCAGAUGUG (SEQ ID NO: 16).
>hsa-miR-514a-3p MIMAT0002883
   AUUGACACUUCUGUGAGUAGA (SEQ ID NO: 17).
>hsa-miR-92b-5p MIMAT0004792
   AGGGACGGGACGCGGUGCAGUG (SEQ ID NO: 18).
>hsa-miR-27a-5p MIMAT0004501
   AGGGCUUAGCUGCUUGUGAGCA (SEQ ID NO: 19).
>hsa-miR-6873-3p MIMAT0027647
   UUCUCUCUGUCUUUCUCUCUCAG (SEQ ID NO: 20).
>hsa-miR-451a MIMAT0001631
   AAACCGUUACCAUUACUGAGUU (SEQ ID NO: 21).
>hsa-miR-122-5p MIMAT0000421
   UGGAGUGUGACAAUGGUGUUUG (SEQ ID NO: 22).
>hsa-miR-122b-3p MIMAT0019877
   AAACACCAUUGUCACACUCCAC (SEQ ID NO: 23).

The target biomarkers are preferably mature miRNA, but may also be a precursor (e.g., pri-miRNA and pre-miRNA).

Of the target biomarkers, miR-204-3p, miR-1468-5p, miR-424-5p, miR-16-2-3p, miR-30e-5p, miR-1246, miR-1290, miR-378a-3p, miR-483-5p, miR-146a-5p, miR-202-5p, miR-370-3p, miR-506-3p, miR-3184-3p, miR-423-5p, miR-365a-5p, miR-514a-3p, miR-92b-5p, miR-27a-5p, and miR-6873-3p are preferable, miR-204-3p, miR-1468-5p, miR-424-5p, miR-16-2-3p, miR-30e-5p, miR-1246, miR-1290, miR-378a-3p, and miR-483-5p are more preferable, and miR-204-3p is even more preferable, from the viewpoint of being more suitable for determining the likelihood of pregnancy and/or the likelihood of childbirth resulting from the pregnancy.

Of the target biomarkers, miR-451a, miR-202-5p, miR-3184-3p, miR-423-5p, miR-514a-3p, miR-122-5p, miR-122b-3p, and miR-483-5p are preferable from the viewpoint of high levels of expression and excellence as detection targets.

The number of target biomarkers to be detected in step (1) may be one or more. From the viewpoint of test accuracy and other factors, the number of target biomarkers is preferably 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 21 or more, 22 or more, or all types (23).

In one aspect of the present invention, the at least one target biomarker detected in step (1) preferably includes at least one miRNA selected from the group consisting of miR-16-2-3p, miR-378a-3p, miR-483-5p, miR-1246, and miR-1290, more preferably includes at least one miRNA selected from the group consisting of miR-16-2-3p, miR-378a-3p, and miR-483-5p, and most preferably includes miR-16-2-3p, miR-378a-3p, and miR-483-5p, from the viewpoint of test accuracy and being particularly suitable for measurement by using a simpler method (e.g., PCR) (for example, biomarkers that can be stably detected).

The detection is typically performed by measuring the level or concentration of the target biomarkers. The concentration is not limited to absolute concentration, and may also be a relative concentration, weight per unit volume, or raw data measured to determine an absolute concentration.

The method for detecting target biomarkers is not particularly limited as long as the method can specifically detect some or all of the target biomarkers. Specific detection methods include, for example, RNA-seq analysis, RT-PCR, nucleic-acid chip analysis, and northern blotting.

For RNA-seq analysis, specifically, the following method can be given as an example: cDNA is prepared from RNA derived from a subject according to a commonly used method, and sequence analysis is performed using next-generation sequencing or the like, followed by mapping, gene expression analysis, expression level analysis, or the like based on the obtained data to obtain information of expression levels.

For RT-PCR, specifically, the following method can be given as an example: cDNA is prepared from RNA derived from a subject according to a commonly used method, and then a pair of primers (a forward strand that binds to the cDNA (- strand) and a template strand that binds to the + strand) are hybridized to the cDNA so that the target region can be amplified using the cDNA as a template, followed by performing PCR according to a commonly used method to detect the resulting amplified double-stranded DNA. The amplified double-stranded DNA can be detected, for example, by using a method of detecting labeled double-stranded DNA produced by performing the PCR described above by using primers labeled in advance with RI or fluorescent substances, or a method of transferring the produced double-stranded DNA to a nylon membrane or similar material according to a commonly used method to allow it to hybridize with a labeled probe, and detecting the hybridized product.

For nucleic-acid chip analysis, the following method can be given as an example: a nucleic acid chip with (single-stranded or double-stranded) a nucleic acid probe attached is prepared, and this is hybridized with RNA derived from a subject or a nucleic acid prepared from the RNA according to a commonly used method to detect the formed double-strand.

For northern blotting, specifically, the following method can be given as an example: a probe is labeled with a radioactive isotope (such as 32P or 33P: RI) or a fluorescent substance, and hybridized with RNA derived from a subject, followed by measuring the formed double strand by detecting signals originating from the label of the probe (labeling substances such as RI or a fluorescent substance) using a radiation detector or fluorescence detector.

The testing method of the present invention including step (1) can provide the level and/or concentration of the target biomarkers, which are indicators for determining the likelihood of pregnancy from an egg that had been contained in follicular fluid and/or the likelihood of childbirth resulting from the pregnancy, thereby assisting in this determination.

The testing results from the testing method including step (1) of the present invention can be used in determining whether to fertilize a collected egg, whether to cryopreserve a collected egg, whether to thaw and fertilize a cryopreserved egg, whether to cryopreserve a fertilized egg, and which fertilized eggs to thaw and implant.

### 1-2. Step (2)

In one aspect, the testing method of the present invention preferably further includes step (2) of determining the likelihood of pregnancy from the egg and/or the likelihood of childbirth resulting from the pregnancy on the basis of the level or concentration of the miRNA detected in step (1).

Step (2) more specifically includes at least one step selected from the group consisting of step (2a) and step (2b): wherein
step (2a) determines that the likelihood of pregnancy from the egg and/or the likelihood of childbirth resulting from the pregnancy is high when the level or concentration of at least one miRNA selected from the group consisting of miR-1468-5p, miR-424-5p, miR-16-2-3p, miR-30e-5p, miR-146a-5p, miR-202-5p, and miR-451a detected in step (1) is equal to or higher than a cutoff value, and/or determining that the likelihood of pregnancy from the egg and/or the likelihood of childbirth resulting from the pregnancy is low when the level or concentration is equal to or lower than the cutoff value; and
step (2b) determines that the likelihood of pregnancy from the egg and/or the likelihood of childbirth resulting from the pregnancy is high when the level or concentration of at least one miRNA selected from the group consisting of miR-204-3p, miR-1246, miR-1290, miR-378a-3p, miR-483-5p, miR-370-3p, miR-506-3p, miR-3184-3p, miR-423-5p, miR-365a-5p, miR-514a-3p, miR-92b-5p, miR-27a-5p, miR-6873-3p, miR-122-5p, and miR-122b-3p detected in step (1) is equal to or lower than a cutoff value, and/or determining that the likelihood of pregnancy from the egg and/or the likelihood of childbirth resulting from the pregnancy is low when the level or concentration is equal to or higher than the cutoff value.

When the likelihood of pregnancy and/or childbirth is determined to be high, steps of assisted reproductive technology can be performed, such as the steps of fertilizing a collected egg, cryopreserving a collected egg, thawing and fertilizing a cryopreserved egg, and transferring a cultured embryo. The method of assisted reproductive technology is not particularly limited, and can be a method according to or based on a known method.

The cutoff value can be appropriately set by those skilled in the art from viewpoints such as sensitivity, specificity, positive predictive value, and negative predictive value. For example, the cutoff value can be a value determined each time or a predetermined value based on the level and/or concentration of the target biomarkers in various samples of follicular fluid (e.g., a sample of follicular fluid of an egg that resulted in pregnancy and a sample of follicular fluid of an egg that did not result in pregnancy). More specifically, for example, the cutoff value can be set by measuring the level or concentration of the target biomarkers in the samples of follicular fluid and performing statistical analysis based on receiver operating characteristic (ROC) curve analysis using the measured values (more specifically, a method using the Youden index is given as an example). The cutoff value can be, for example, any percentile value of the level or concentration of a target protein in a sample of follicular fluid from a reference subject group, such as any value between the 10th to 90th percentile, any value between the 30th to 70th percentile, or any value between the 40th to 60th percentile. Once established, the database of the evaluation population may be used to set the cutoff value without any changes. Alternatively, the database of the evaluation population may be updated as appropriate by incorporating new subjects, including the subjects of the present invention, into the evaluation population, and used to set the cutoff value.

### 2. Drug for Testing Target Disease

In one aspect, the present invention relates to a drug for testing the likelihood of pregnancy from an egg of a subject and/or the likelihood of childbirth resulting from the pregnancy, including a detection agent for a target biomarker (which may also be referred to as "the detection agent of the present invention" in the present specification). (The drug may also be referred to as "the testing drug of the present invention" in the present specification). The following explains the testing drug of the present invention.

The target biomarkers, target diseases, etc. are as defined in the "1. Testing Method" section above.

The detection agent of the present invention is not particularly limited as long as the detection agent can specifically detect the target biomarkers. Examples of such detection agents include primers and probes for the target biomarkers.

The detection agent of the present invention may be modified as long as its function is not significantly impaired. Examples of modifications include the addition of labels, such as fluorescent dyes, enzymes, proteins, radioisotopes, chemiluminescent substances, or biotin.

The fluorescent dyes suitably used in the present invention are those generally used for labeling nucleotides to detect and quantify nucleic acids. Examples of fluorescent dyes include, but are not limited to, HEX (4,7,2',4',5',7'-hexachloro-6-carboxylfluorescein, a green fluorescent dye), fluorescein, NED (trade name, manufactured by Applied Biosystems, a yellow fluorescent dye), 6-FAM (trade name, manufactured by Applied Biosystems, a yellow-green fluorescent dye), and rhodamine or its derivatives (e.g., tetramethylrhodamine (TMR)). The method for labeling a nucleotide with a fluorescent dye can be suitably selected from known labeling methods (see Nature Biotechnology, 14, 303-308 (1996)). Additionally, commercially available fluorescence labeling kits can also be used (e.g., Oligonucleotide ECL 3'-Oligolabeling System, manufactured by Amersham Pharmacia).

The detection agent of the present invention can also be immobilized on any solid phase for use. Thus, the testing drug of the present invention can be provided in the form of a substrate on which the detection agent is immobilized (e.g., a microarray chip with immobilized probes).

The solid phase used for immobilization is not particularly limited as long as polynucleotides or the like can be immobilized on the solid phase. Examples of solid phases include glass plates, nylon membranes, microbeads, silicon chips, capillaries, and other substrates. The immobilization of the detection agent on a solid phase is not particularly limited. Immobilization methods according to the type of immobilized probes are well known in the art; for example, in the case of a microarray, a commercially available spotting device (e.g., Amersham) can be used (e.g., in situ synthesis of oligonucleotides by photolithographic technology (Affymetrix) or inkjet technology (Rosetta Inpharmatics)).

The primers and probes are not particularly limited as long as they selectively (specifically) recognize the target biomarkers, nucleic acids derived from the target biomarkers, etc. As used herein, the phrase "selectively (specifically) recognize" means, for example, specifically detecting a target biomarker in northern blotting analysis, or specifically amplifying a target biomarker or a nucleic acid (such as cDNA) derived from the target biomarker in RT-PCR. However, the meaning of the phrase is not limited to these, and the phrase can be interpreted as indicating any method that allows those skilled in the art to determine that a detected or amplified product is derived from a target biomarker.

Specific examples of primers, probes, etc. can be at least one selected from the group consisting of the following polynucleotides listed in (a) and the polynucleotides listed in (b):
(a) a polynucleotide containing at least 15 consecutive bases of the base sequence of a target biomarker and/or a polynucleotide complementary to the polynucleotide; and
(b) a polynucleotide containing at least 15 bases that hybridizes under stringent conditions to the base sequence of a target biomarker or a complementary base sequence thereof.

The "complementary polynucleotide" or "complementary base sequence" (complementary strand, template strand) refers to a polynucleotide or base sequence that is basally complementary to the full-length sequence of a polynucleotide composed of the base sequence of a target biomarker, or its partial sequence having at least 15 consecutive bases of the base sequence (both of which are referred to here as "coding strand" for convenience), based on base pair relationships, such as A:T and G:C. However, the complementary strand is not limited to a strand that forms a full complementary sequence with the base sequence of the target coding strand, but may also be a strand that has a complementary relationship to the extent that it can hybridize with the target coding strand under stringent conditions. Stringent conditions can be determined based on the melting temperature (Tm) of the nucleic acid to which a complex or probe binds, as taught by Berger and Kimmel (1987, Guide to Molecular Cloning Techniques, Methods in Enzymology, Vol. 152, Academic Press, San Diego, CA). For example, the washing conditions after hybridization can be typically conditions such as 1× SSC, 0.1% SDS, 37°C. It is preferred that the complementary strand remains hybridized with the target coding strand even when washed under such conditions. Although there is no particular limitation, washing conditions can the following: more stringent hybridization conditions of about 0.5× SSC, 0.1% SDS, 42°C, and even more stringent hybridization conditions of about 0.1× SSC, 0.1% SDS, 65°C. Specifically, the complementary strand can be, for example, a strand composed of a base sequence that is fully complementary to the base sequence of the target coding strand, or a strand composed of a base sequence having at least 90%, preferably 95%, more preferably at least 98%, and even more preferably at least 99% identity with the strand.

The primers, probes, etc. can be designed based on the base sequence of a target biomarker, for example, by using various design programs. Specifically, candidate primer or probe sequences obtained by inputting the base sequence of the target biomarker into a design program, or sequences containing at least part of the sequences, can be used as primers or probes.

The base length of primers, probes, etc. is not particularly limited as long as they have a length of at least 15 consecutive bases as described above, and can be appropriately set according to the intended use. The base length is, for example, 15 to 35 bases when the detection agent is used as a primer, and, for example, 15 to 35 bases when the detection agent is used as a probe.

The testing drug of the present invention may contain other detection agents (e.g., probes for detecting nucleic acids, such as other miRNAs, or antibodies) in addition to the detection agent of the present invention. In this case, the testing drug of the present invention may be able to examine other diseases or conditions in addition to the target disease. In this case, the detection agent of the present invention is included as a detection agent for testing a target disease. From this perspective, the testing drug of the present invention, in one aspect, is a testing drug for a target disease, containing a detection agent for testing the target disease containing the detection agent of the present invention.

The testing drug of the present invention may be in the form of a composition. The composition may optionally contain other components. Other components include, for example, bases, carriers, solvents, dispersants, emulsifiers, buffers, stabilizers, excipients, binders, disintegrants, lubricants, thickeners, moisturizers, colorants, fragrances, and chelating agents.

The testing drug of the present invention may be in the form of a kit. In addition to the detection agent or composition containing the detection agent described above, the kit may contain items that can be used for detecting the target biomarkers in body fluid of a subject. Specific examples of such items include various reagents (e.g., buffer solutions) and instruments (e.g., devices for purifying and separating body fluids).

### Examples

The following provides a detailed explanation of the present invention based on Examples. However, the present invention is not limited to these Examples.

### Test Example 1: Search for Markers to Test the Likelihood of Pregnancy and/or the Likelihood of Childbirth from the Pregnancy Research Subjects

Samples of follicular fluid (FF) were collected from women undergoing assisted reproductive technology (ART) at a hospital. Informed consent was obtained from each patient before ovarian stimulation. Small RNA sequencing analysis was performed on the follicular fluid (FF) from cases in which pregnancy occurred from eggs retained in the FF (pregnancy cases, N = 10) and cases in which pregnancy did not occur from eggs retained in the FF (non-pregnancy cases, N = 14). For non-pregnancy examples, cases were selected in which the maternal age and grade of the transferred embryos were similar to those of pregnancy cases. All pregnancy cases resulted in full-term live births.

### Method of Ovarian Stimulation, In Vitro Fertilization, and Microinsemination

Ovarian stimulation involved administering urinary follicle-stimulating hormone (FSH) or recombinant FSH at 150 to 300 IU per day for the first two days, after which the doses were adjusted individually based on the follicular response under gonadotropin-releasing hormone antagonist or agonist protocols. At the point that the mean diameter of the follicles reached at least 18 mm, 10,000 IU of human chorionic gonadotropin was administered. After 35.5 hours, oocyte retrieval was performed. The protocols used for oocyte retrieval and preparation, sperm preparation, and in vitro fertilization (IVF)/intracytoplasmic sperm injection (ICSI) have been previously reported in Gynecol Endocrinol, 2010; 26(7): 494-9, doi:10.3109/09513591003632050.

### Sample Collection

Follicular fluids (FF) were collected from follicles with a diameter of 18 to 25 mm from the patients. An oocyte and FF were collected individually. All FF samples were placed individually in 15 mL conical tubes and centrifuged at 15,000 rpm for 10 minutes. The clear supernatant was aliquoted into 2 mL tubes and stored at -80°C until further analysis.

### EV Isolation

About 1 mL of each sample was centrifuged at 10,000 g for 40 minutes at 4°C using an ultracentrifuge (Kubota Corporation, Model 7000). The supernatant was filtered using a 0.22 µm filter (Millex-GV 33 mm, Millipore) and then ultracentrifuged at 110,000 g at 4°C for 70 minutes using an MLS50 rotor (Beckman Coulter Inc., USA). The pellets were washed with PBS, ultracentrifuged under the same conditions, and resuspended in PBS to extract small EVs. The extracted small EVs were subjected to western blotting to confirm whether sEV markers (CD9, CD63, CD81) were expressed. The particle size distribution was also confirmed to have peaked around 100 nm.

### Determination of Base Sequence of Small RNA

RNA was extracted using a miRNeasy Plus Mini Kit (Qiagen, Hilden, Germany). The total RNA concentration of each sample was measured using a Qubit RNA HS Assay Kit (Thermo Fisher Scientific, Waltham, MA). Small RNA libraries were prepared using a NEBNext Multiplex Small RNA Library Prep Set for Illumina (New England Biolabs, Ipswich, MA), and index codes were added to attribute sequences to each sample. Subsequently, the PCR products were purified using a QIAquick PCR Purification Kit (Qiagen) and 6% TBE gel (120 V, 60 minutes). Furthermore, DNA fragments corresponding to 140-160 bp (the length of small noncoding RNA with the 3' and 5' adaptors) were recovered, and the concentration of complementary DNAs was measured using a Qubit dsDNA HS Assay Kit and a Qubit 2.0 Fluorometer (Life Technologies, Carlsbad, CA). Finally, single-end reads were obtained using an Illumina MiSeq or NextSeq (Illumina, San Diego, CA).

### Bioinformatics Analysis

The raw data files of small RNA sequencing were analyzed with a CLC Genomics Workbench version 9.5.3 program (Qiagen). After adapter trimming, the data were mapped to the miRbase 22 database, allowing up to two mismatches, and normalized using the count of reads per million mapped reads. Subsequently, RStudio (RStudio, Boston, MA) and R software (ver. 4.0.3) were used. To visualize the volcano plots, the log2 fold change (log2 FC) and adjusted p-values for each gene were calculated using the Wald test in DESeq2 (ver. 1.30.0). After excluding miRNAs with maximum reads per million (RPM) of <100, 365 miRNAs were selected for subsequent analysis. miRNAs in FF-EVs that were differentially expressed between pregnancy and non-pregnancy cases were selected using the t-test with an adjusted p-value <0.05 and log2 FC >0.7 as the cutoff criteria. The results indicated that 7 miRNAs were highly expressed in pregnancy cases, while 16 miRNAs were highly expressed in non-pregnancy cases. Additionally, to examine the diagnostic capability of miRNAs, a receiver operating characteristic (ROC) curve was created, and the area under the curve (AUC) was also determined.

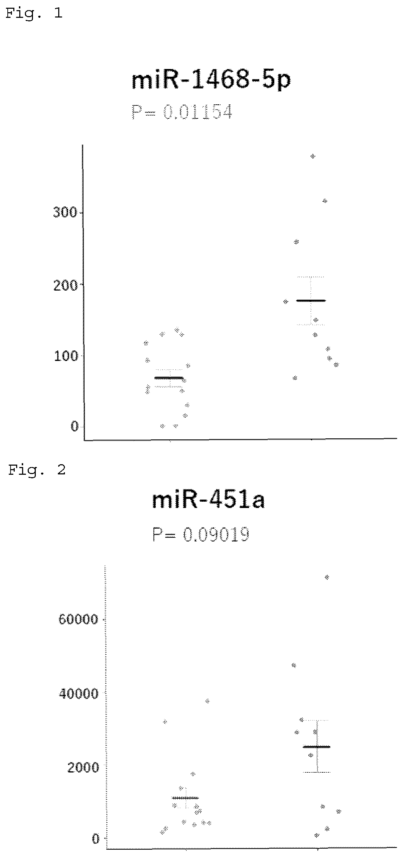

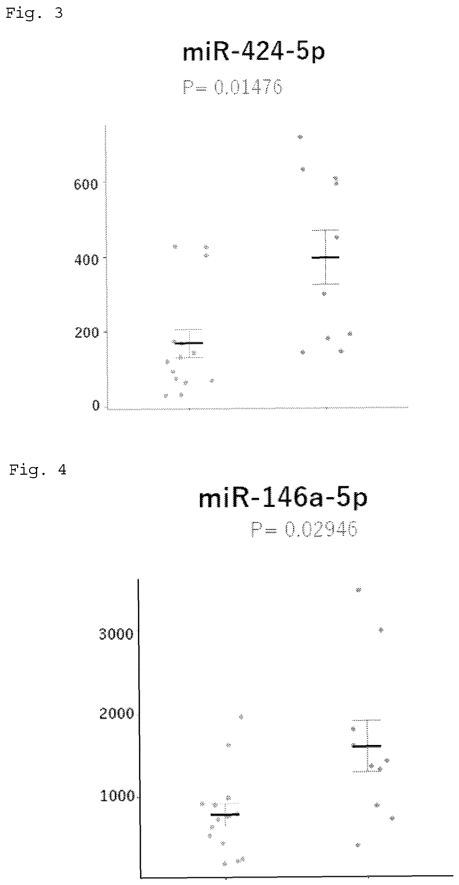

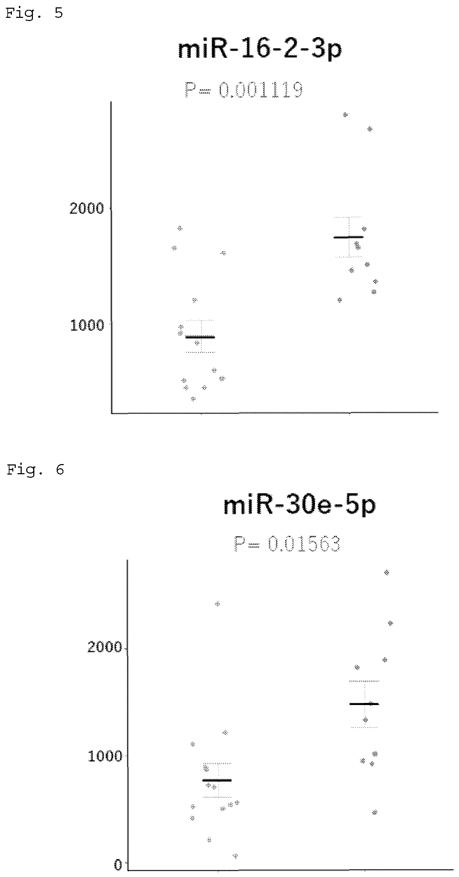

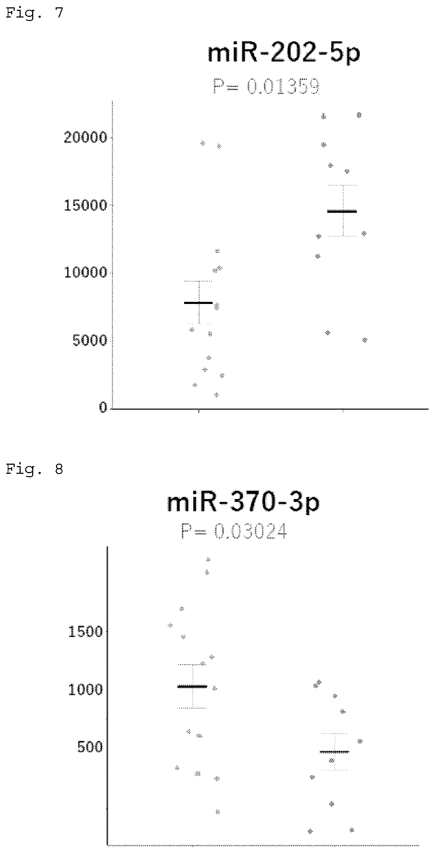

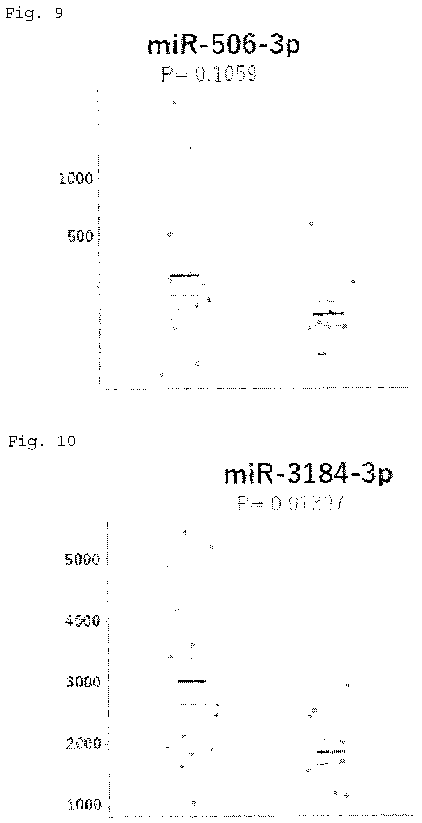

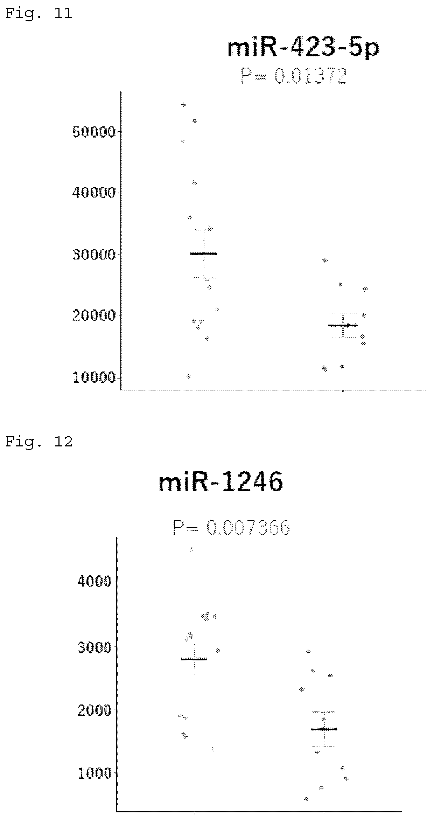

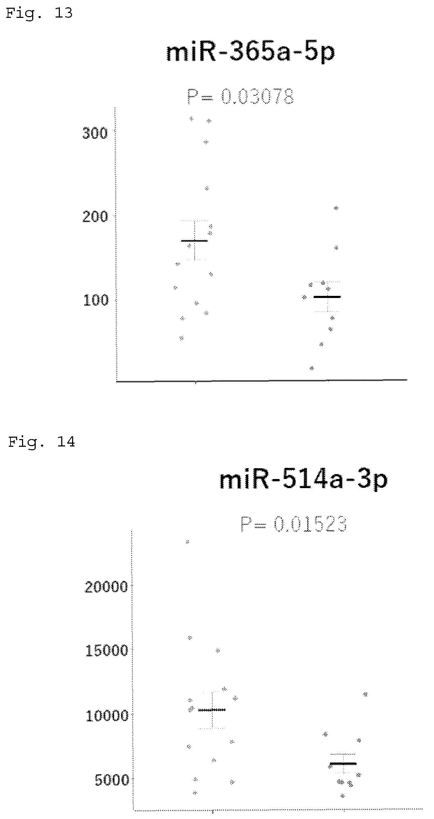

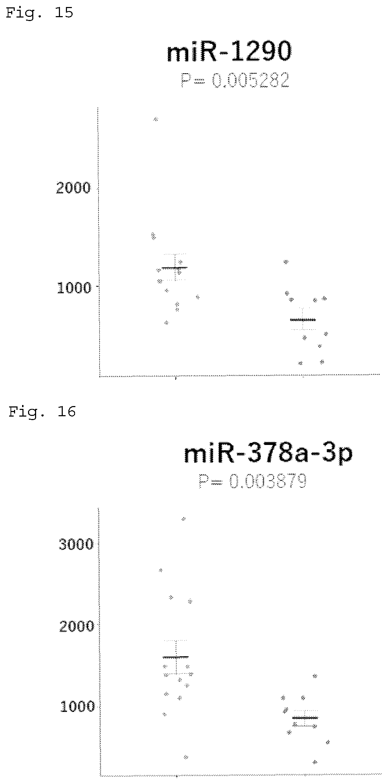

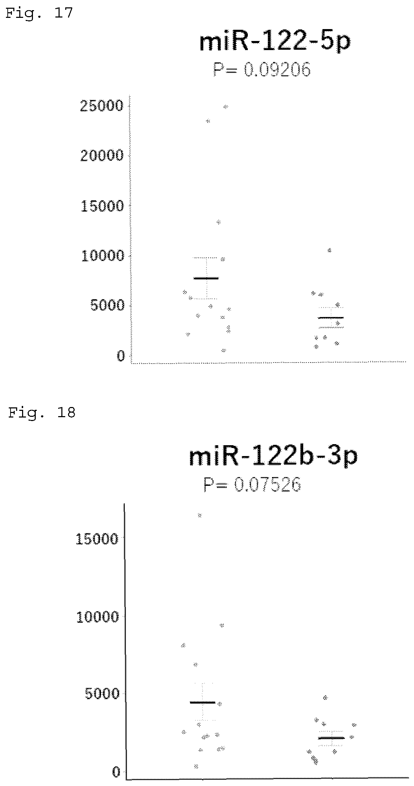

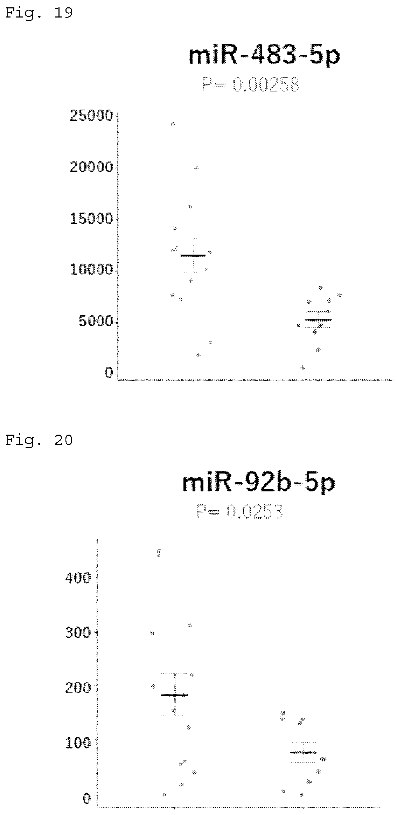

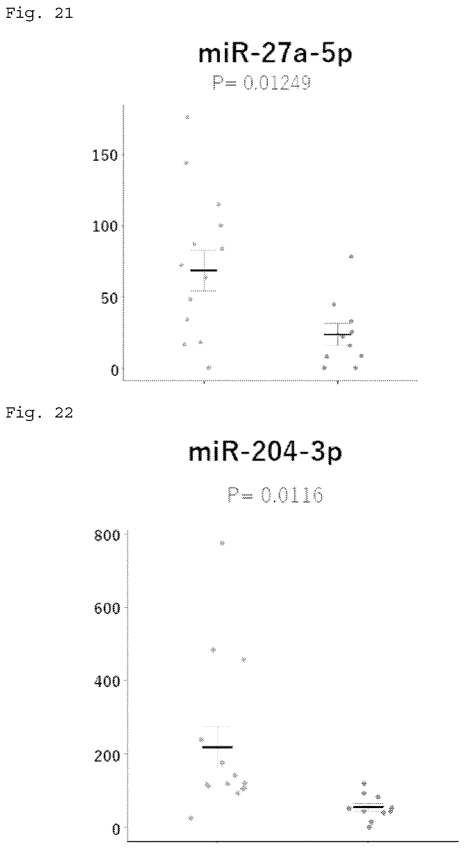

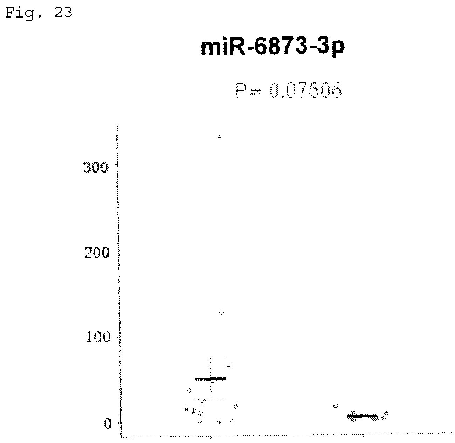

Figs. 1 to 23 show the read counts of the selected marker miRNAs in pregnancy and non-pregnancy cases.

Table 1 shows the AUC. In Table 1, "P up" indicates miRNA with an increased expression level in pregnancy cases as compared to non-pregnancy cases, while "P down" indicates miRNA with a decreased expression level in pregnancy cases as compared to non-pregnancy cases.

**Table 1**

| | AUC | |
|---|---|---|
| miR-1468-5p | 0.8393 | P up |
| miR-451a | 0.6643 | P up |
| miR-424-5p | 0.8607 | P up |
| miR-146a-5p | 0.7643 | P up |
| miR-16-2-3p | 0.8714 | P up |
| miR-30e-5p | 0.8214 | P up |
| miR-202-5p | 0.7929 | P up |
| miR-370-3p | 0.7357 | P down |
| miR-506-3p | 0.7036 | P down |
| miR-3184-3p | 0.75 | P down |
| miR-423-5p | 0.75 | P down |
| miR-1246 | 0.8357 | P down |
| miR-365a-5p | 0.7321 | P down |
| miR-514a-3p | 0.7643 | P down |
| miR-1290 | 0.8357 | P down |
| miR-378a-3p | 0.8679 | P down |
| miR-122-5p | 0.6714 | P down |
| miR-122b-3p | 0.6857 | P down |
| miR-483-5p | 0.85 | P down |
| miR-92b-5p | 0.7107 | P down |
| miR-27a-5p | 0.7607 | P down |
| miR-204-3p | 0.9036 | P down |
| miR-6873-3p | 0.7786 | P down |

Furthermore, multiple miRNAs were combined to calculate a new variable (predictive probability), and an ROC was created in the same manner as described above. Specifically, the procedure was as follows: using the glm function of R software, logistic regression analysis was performed for pregnancy and non-pregnancy based on the expression levels of miR-16-2-3p, miR-378a-3p, and miR-483-5p, and the predictive probability was calculated. The results indicated that the AUC from the combination of miR-16-2-3p, miR-378a-3p, and miR-483-5p was 0.9643.

## Claims

1. A method of testing the likelihood of pregnancy from an egg that had been contained in a follicular fluid collected from a subject and/or the likelihood of childbirth resulting from the pregnancy, comprising step (1) of detecting at least one miRNA selected from (a) the group consisting of miR-16-2-3p, miR-378a-3p, miR-483-5p, miR-1246, and miR-1290, and/or from (b) the group consisting of miR-204-3p, miR-1468-5p, miR-424-5p, miR-30e-5p, miR-146a-5p, miR-202-5p, miR-370-3p, miR-506-3p, miR-3184-3p, miR-423-5p, miR-365a-5p, miR-514a-3p, miR-92b-5p, miR-27a-5p, miR-6873-3p, miR-451a, miR-122-5p, and miR-122b-3p, in a sample of the follicular fluid.

2. The method according to claim 1, wherein the miRNA includes at least one miRNA selected from the group consisting of miR-16-2-3p, miR-378a-3p, miR-483-5p, miR-1246, and miR-1290.

3. The method according to claim 1, wherein the miRNA includes at least one miRNA selected from the group consisting of miR-16-2-3p, miR-378a-3p, and miR-483-5p.

4. The method according to claim 1, wherein the miRNA includes miR-16-2-3p, miR-378a-3p, and miR-483-5p.

5. The method according to claim 1, wherein the sample of the follicular fluid is a cell-reduced fraction of the follicular fluid.

6. The method according to claim 1, wherein the sample of the follicular fluid is an extracellular vesicle purified from the follicular fluid.

7. The method according to claim 1, wherein the miRNA includes at least one miRNA selected from the group consisting of miR-204-3p, miR-1468-5p, miR-424-5p, miR-16-2-3p, miR-30e-5p, miR-1246, miR-1290, miR-378a-3p, miR-483-5p, miR-146a-5p, miR-202-5p, miR-370-3p, miR-506-3p, miR-3184-3p, miR-423-5p, miR-365a-5p, miR-514a-3p, miR-92b-5p, miR-27a-5p, and miR-6873-3p.

8. The method according to claim 1, further comprising step (2) of determining the likelihood of pregnancy from the egg and/or the likelihood of childbirth resulting from the pregnancy on the basis of a level or concentration of the miRNA detected in step (1).

9. The method according to claim 8,
wherein
step (2) comprises at least one step selected from the group consisting of step (2a) and step (2b),
wherein
step (2a) determines that the likelihood of pregnancy from the egg and/or the likelihood of childbirth resulting from the pregnancy is high when the level or concentration of at least one miRNA selected from the group consisting of miR-1468-5p, miR-424-5p, miR-16-2-3p, miR-30e-5p, miR-146a-5p, miR-202-5p, and miR-451a detected in step (1) is equal to or higher than a cutoff value, and/or determines that the likelihood of pregnancy from the egg and/or the likelihood of childbirth resulting from the pregnancy is low when the level or concentration is equal to or lower than the cutoff value, and
step (2b) determines that the likelihood of pregnancy from the egg and/or the likelihood of childbirth resulting from the pregnancy is high when the level or concentration of at least one miRNA selected from the group consisting of miR-204-3p, miR-1246, miR-1290, miR-378a-3p, miR-483-5p, miR-370-3p, miR-506-3p, miR-3184-3p, miR-423-5p, miR-365a-5p, miR-514a-3p, miR-92b-5p, miR-27a-5p, miR-6873-3p, miR-122-5p, and miR-122b-3p detected in step (1) is equal to or lower than a cutoff value, and/or determines that the likelihood of pregnancy from the egg and/or the likelihood of childbirth resulting from the pregnancy is low when the level or concentration is equal to or higher than the cutoff value.

10. The method according to any one of claims 1 to 9, wherein the pregnancy is pregnancy through assisted reproductive technology.

11. The method according to any one of claims 1 to 9, wherein the subject is a human.

12. A drug for testing the likelihood of pregnancy from an egg of a subject and/or the likelihood of childbirth resulting from the pregnancy, comprising a detection agent for at least one miRNA selected from (a) the group consisting of miR-16-2-3p, miR-378a-3p, miR-483-5p, miR-1246, and miR-1290, and/or from (b) the group consisting of miR-204-3p, miR-1468-5p, miR-424-5p, miR-30e-5p, miR-146a-5p, miR-202-5p, miR-370-3p, miR-506-3p, miR-3184-3p, miR-423-5p, miR-365a-5p, miR-514a-3p, miR-92b-5p, miR-27a-5p, miR-6873-3p, miR-451a, miR-122-5p, and miR-122b-3p.
